(19) Europäisches Patentamt
European Patent Office
Office européen des brevets

(11) Numéro de publication: 0 337 858 B1

(12) # FASCICULE DE BREVET EUROPEEN

(45) Date de publication de fascicule du brevet: **08.06.94**

(51) Int. Cl.5: **C07D 491/048**, A61K 31/435, //(C07D491/048,307:00,221:00)

(21) Numéro de dépôt: **89400956.2**

(22) Date de dépôt: **06.04.89**

(54) **Procédé stéréospécifique pour la préparation des enantiomères de la furo[3,4-c]pyridine, composés ainsi obtenus et compositions thérapeutiques à base de ces composés.**

(30) Priorité: **06.04.88 GB 8808001**

(43) Date de publication de la demande: **18.10.89 Bulletin 89/42**

(45) Mention de la délivrance du brevet: **08.06.94 Bulletin 94/23**

(84) Etats contractants désignés: **ES GR IT**

(56) Documents cités:
**EP-A- 0 204 269**
**GB-A- 2 092 586**
**GB-A- 2 137 618**

**TETRAHEDRON LETTERS, no. 11, 1976, pages 809-812; E.J. COREY et al.: "A new general method for protection of the hydroxyl function"**

**JOURNAL OF THE CHEMICAL SOCIETY, CHEMICAL COMMUNICATIONS, 1980, pages 1026-1027; S. TERASHIMA et al.: "Asymmetric reduction of alpha-beta-unsaturated ketones with lithium aluminium hydride partially decomposed by (-)-N-methylephedrine and N-ethylaniline"**

(73) Titulaire: **SOCIETE DE CONSEILS DE RECHERCHES ET D'APPLICATIONS SCIENTIFIQUES (S.C.R.A.S.)**
**51/53 rue du Docteur Blanche**
**F-75016 Paris(FR)**

(72) Inventeur: **Bonato, Marc**
**Mas de la Pomparesse**
**F-30390 Aramon(FR)**
Inventeur: **Eck, Charles**
**191 Maple Avenue**
**Schrewsbury, Massachusetts 01545(US)**

(74) Mandataire: **Lachassagne, Jacques**
**Boite Postale 07**
**F-78430 Louveciennes (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 337 858 B1

Rank Xerox (UK) Business Services
(3.10/3.09/3.3.3)

## Description

La présente invention concerne des procédés stéréospécifiques pour la préparation des énantiomères (+) de la furo [3,4-c] pyridine substituée en position 3, répondant à la formule générale I:

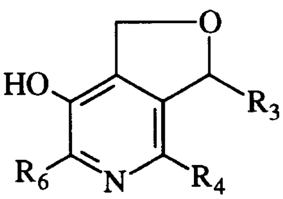

I

dans laquelle
$R_3$ représente un radical hydrocarboné, saturé ou non saturé, en chaîne droite ou ramifiée, comportant de 1 à 5 atomes de carbone, un groupement hétérocyclique ayant jusqu'à 6 atomes de carbone dans le cycle, un groupement phényle ou cyclohexyle, un groupement alcoylphényle ou alcénylphényle, chacun de ces groupements étant éventuellement substitué par un ou plusieurs atomes de chlore, de brome ou de fluore, un ou plusieurs groupements trifluorométhyle, alcoyle ayant de 1 à 5 atomes de carbone, alcoxy ayant de 1 à 5 atomes de carbone, alcoylthio ayant de 1 à 5 atomes de carbone, dialcoylamino dans lequel chacun des groupements alcoyle comprend de 1 à 5 atomes de carbone, dialcoylaminoalcoxy dans lesquels chacun des deux groupements alcoyle et le groupement alcoxy contient de 1 à 5 atomes de carbone ou un groupement $\alpha$- ou $\beta$-alcoxy-N-pyrrolidinyle dans lequel l'alcoxy contient de 1 à 5 atomes de carbone;
$R_4$ représente un atome d'hydrogène ou d'halogène et
$R_6$ représente un groupememt alcoyle ou alcényle en chaîne droite ou ramifiée ayant jusqu'à 6 atomes de carbone, éventuellement substitué par un radical hydroxy, cyano, amino ou un radical amino substitué ou par un radical alcoyle ou alcényle ayant jusqu'à 4 atomes de carbone.

La présente invention concerne également les composés ainsi obtenus, soit sous forme d'énantiomères (+) purs, soit sous forme de mélanges d'énantiomères dans lesquels l'énantiomère (+) est largement prédominant.

Des familles de ces dérivés ainsi que les procédés pour leur obtention ont déjà été décrits dans les précédents brevets espagnols Nos. 509 449, 531 285, 536 814, 537 666, 537 665, 540 080, 546 590, 546 702, 548 209, les brevets grecs Nos. 75 905 et 79 577 et les brevets italiens Nos. 1 157 252, 1 173 770, 1 176 986, 1 177 185, 1 188 188, 1 190 413 et demandes de brevets Nos. 23565A84, 19327A85, 22035A85. Toutefois, ces procédés ont abouti généralement à des mélanges racémiques desdits composés.

On a découvert que, dans la plupart des cas, l'un des isomères optiques d'un composé spécifique possède une activité thérapeutique plus importante que celle de l'autre isomère. Il est intéressant, pour cette raison, de trouver des procédés pour l'obtention sélective, ou au moins prédominante, d'un isomère spécifique de chacun de ces composés.

L'invention concerne un procédé stéréospécifique pour la préparation du dérivé (+) de la furo [3,4-c] pyridine répondant à la formule générale I, comme défini ci-dessus, le procédé comprenant les étapes suivantes :

(a) Oxydation d'un dérivé racémique de la pyridine répondant à la formule générale II

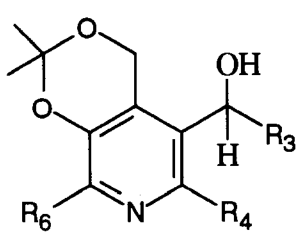

II

dans laquelle $R_3$, $R_4$ et $R_6$ sont tels que définis ci-dessus par un agent oxydant quelconque, tel que, par exemple, du chlorochromate de bipyridinium ou de l'hypochlorite de sodium, dans un solvant organique,

(b) Réduction de la cétone obtenue répondant à la formule générale III

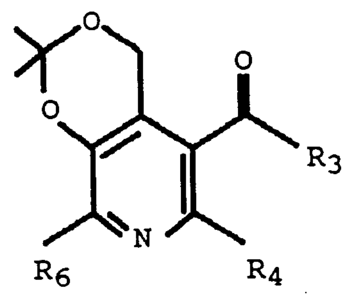

III

dans laquelle $R_3$, $R_4$ et $R_6$ sont tels que définis ci-dessus avec un agent réducteur chiral tel que : B-Ipc-9-BBN (Alpine - borane, Aldrich), N,B - Enantrane (Aldrich), $Ipc_2BCl$ (Aldrich), $BH_3$-AMDPB (2:1) (voir S.Itsuno, J.Chem.Soc., Chem.Comm. 1981, 315), (R,R)-2,5-dimethylborolane (voir S.Masamune, J.Am Chem.Soc. 1986, 108, 7402), N,B - Enantride (Aldrich), $LiBH_4$-DBC-t-BuOH (voir K.Soal, J.Chem. Soc., Chem.Comm. 1984, 413), $NaBH_4$-IBA-DIPGF (voir S.Itsuno, J.Chem.Soc., Perkin Trans.1, 1981, 900), K-Glucoride (voir H.C.Brown, J.Org.Chem. 1986, 51, 1934), $LiAlH_4$-Darvon Alc (voir H.Mosher, J.Am.Chem.Soc., 1972, 94, 9254), $LiAlH_4$-MEP-ArOH (voir J.P.Vigneron, Tetrahedron 1976, 32, 939), $LiAlH_4$-Diamine (voir M.Asami, Heterocycles, 1979, 12, 499), $LiAlH_4$-Aminobutanol (voir T.Sato, Tet.Letters 1982, 23, 4111), Binal H (voir R.Noyori, J.Am.Chem.Soc. 1979, 101, 3129), $LiAlH_4$-DBP-EtOH (voir K.Yamamoto, J.Chem.Soc., Chem. Comm. 1984, 1490), $LiAlH_4$-MEP-NEA (voir K.J. Koga, J.Chem. Soc., Chem.Comm. 1980, 1026), $LiAlH_4$-MEP-EAP (voir S.Terashima, Chem.Letters 1984, 239), TBADH (thermoanaérobium brockii alcohol dehydrogenase, Sigma Chem.Co.), CBS reagent (see E.J. Corey et all. J.Am. Chem.Soc., 1987, 109, 5551), $MDBH_2$ et $MDBCl_2$ (M.Bonato et all. in press) ou un catalyseur approprié pour une hydrogénation asymétrique.

La matière première II est un intermédiaire dans le procédé habituel de préparation des dérivés de la furo[3,4-c] pyridine répondant à la formule générale I.

La réduction de la cétone III par l'agent réducteur choisi peut être conduite, par exemple, dans le tétrahydrofurane ou un mélange approprié d'éthers et d'hydrocarbones, et s'effectue selon le schéma de réaction I suivant, pour donner les alcools énantiomères correspondants, dans lesquels $R_3$, $R_4$ et $R_6$ sont tels que précédemment définis.

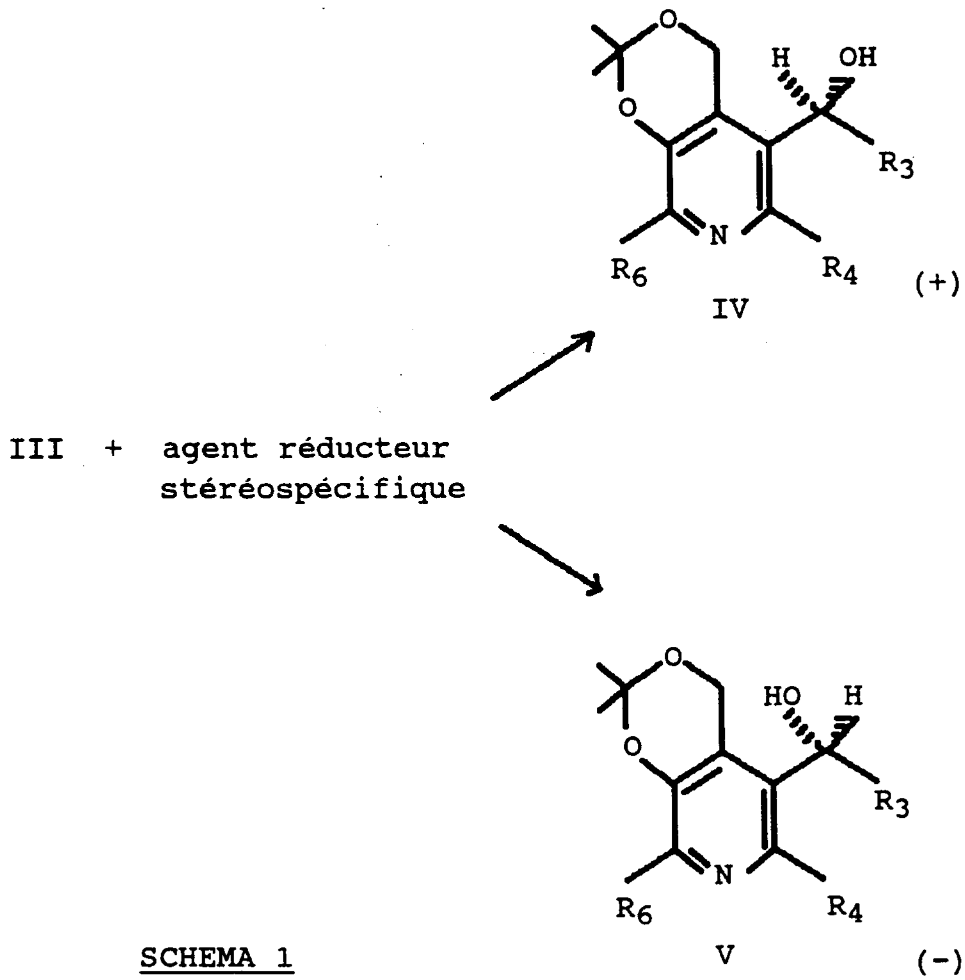


SCHEMA 1

(c) Verrouillage ou blocage stéréospécifique du groupe OH de l'alcool énantiomère (+) ; l'agent de verrouillage peut être un halogène, par exemple, un atome de chlore substitué au groupe OH ; l'agent de blocage peut être constitué par les groupements éther ou ester habituellement utilisés.

(d) Ouverture du cycle acétonide par des acides protiques avec une libération concomitante des groupes $CH_2OH$ et OH sur le cycle pyridine ; dans certains cas, la reprotection des groupes $CH_2OH$ et OH en question peut être nécessaire, soit par acétylation, soit par tosylation ou par toute méthode équivalente.

(e) Cyclisation du composé obtenu et, si nécessaire, déprotection du groupement phénoxy.

Dans une première voie (schéma 2), la réduction de la cétone III par l'agent réducteur choisi, conduit aux composés IV ou V, dont le traitement par un agent de chloration dans un solvant approprié donne des composés de formules générales VI ou VII dans lesquelles $R_3$, $R_4$ et $R_6$ sont tels que définis ci-dessus.

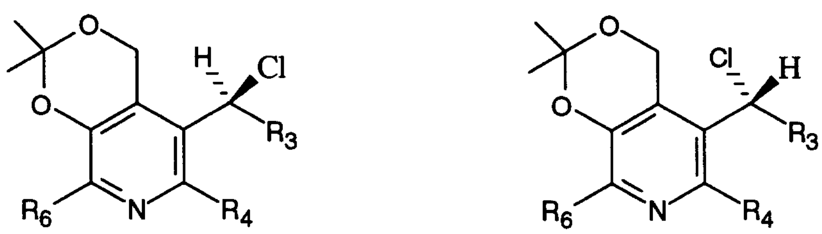

VI VII

L'agent de chloration peut être, par exemple, de la triphénylphosphine avec du tétrachlorure de carbone comme solvant ou du $SO_2Cl_2$ avec du chlorure de méhylène comme solvant ; dans ce dernier cas, une addition de pyridine provoque une inversion de la configuration stéréochimique du dérivé chloro au début

de la séquence de réactions. On peut utiliser les dérivés du brome au lieu des dérivés du chlore avec des résultats équivalents.

La conversion des composés VI et VII en dérivés correspondants de la furo[3,4-c] pyridine est réalisée par clivage du cycle acétonide en milieu acide, suivi d'une cyclisation dans des solvants protiques ou des mélanges de ces derniers à température ambiante ou sous chauffage doux.

La séquence correspondante de réactions est illustrée comme suit.

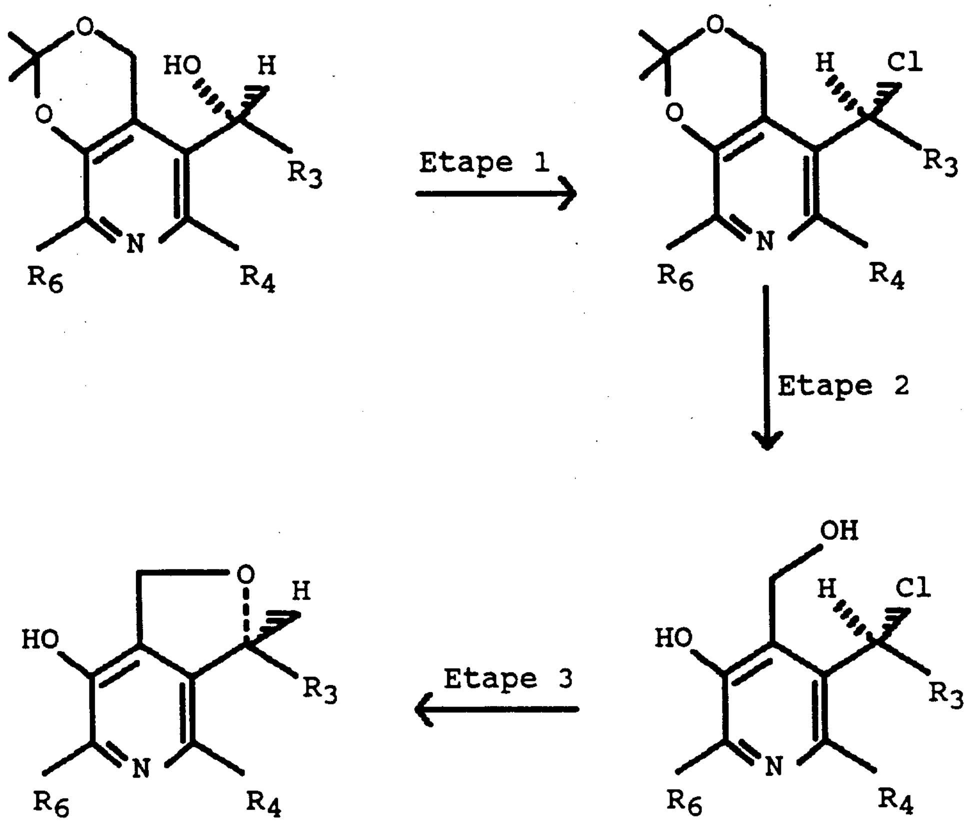


SCHEMA 2
dérivés (-) de la furo[3,4-c]pyridine

Les étapes de la synthèse doivent être conduites avec le plus grand soin, afin d'éviter d'utiliser une méthode qui serait susceptible de racémiser le centre carbinol.

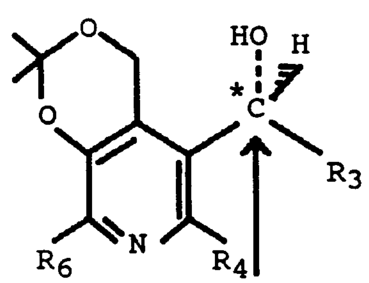

Dans une seconde voie où la configuration du carbone asymétrique des réactifs n'est pas affectée, une première étape consiste à protéger préalablement la fonction alcool secondaire par un groupe stable en milieu acide. On peut utiliser des groupements de protection aboutissant à la formation d'éthers tels que t-butyle diphényle silyle ou méthoxy éthoxy-2 méthyle (MEM) comme décrit par E.J. COREY, J.L. GRAS et P. ULRICH, Tetrahedron Letters, 1976, (11), 809 et S. HANESSIAN et P.LAVALLEE, Can. J. Chem., 1975, 53, 2975, ou aboutissant à la formation d'esters tels que l'acétate, le benzoate et autres esters équivalents. L'ouverture du cycle isopropylidène ou acétonide est réalisée à l'aide des acides protiques ; l'acide trifluoroacétique a été choisi pour cette synthèse.

Les protections de l'alcool en positions 3 et 4' du cycle pyridine sont obtenues respectivement par acétylation et tosylation, dans le cas de la protection à l'éther (schéma 3), ou par tosylation seule (position 4') dans le cas de la protection à l'ester (schéma 4), selon les méthodes traditionnelles généralement utilisées en chimie organique. Lors de l'utilisation d'éthers, une déprotection ultérieure peut être effectuée par divers acides de Lewis du type $TiCl_4$ ou $ZnBr_2$ dans le chlorure de méthylène, comme décrit par E.J. COREY, J.L. GRAS et P.ULRICH, Tetrahedron Letters, 1976, (11), 809 ou du chlorure de triméthyle silyle, de l'iodure de sodium comme décrit par J.H. RIGBY et J.Z. WILSON, Tetrahedron Letters, 1984, (14), 1429. Lorsque des esters sont utilisés, des réactifs basiques de type, par exemple, $MeOH/NH_3$, MeOH/NaOMe, NaOH, $K_2CO_3$ interviennent dans la déprotection ultérieure.

Enfin, la cyclisation et la déprotection du groupement acétyle conduisant au produit final sont obtenues par une attaque de l'alcoolate correspondant sur le carbone 4' portant le groupement de départ (tosyloxy).

Les composés IV et V peuvent être convertis en dérivés correspondants (+) et (-) de la furo[3,4-c]-pyridine ou en mélanges des deux énantiomères, soit à large prédominance (+), soit à large prédominance (-).

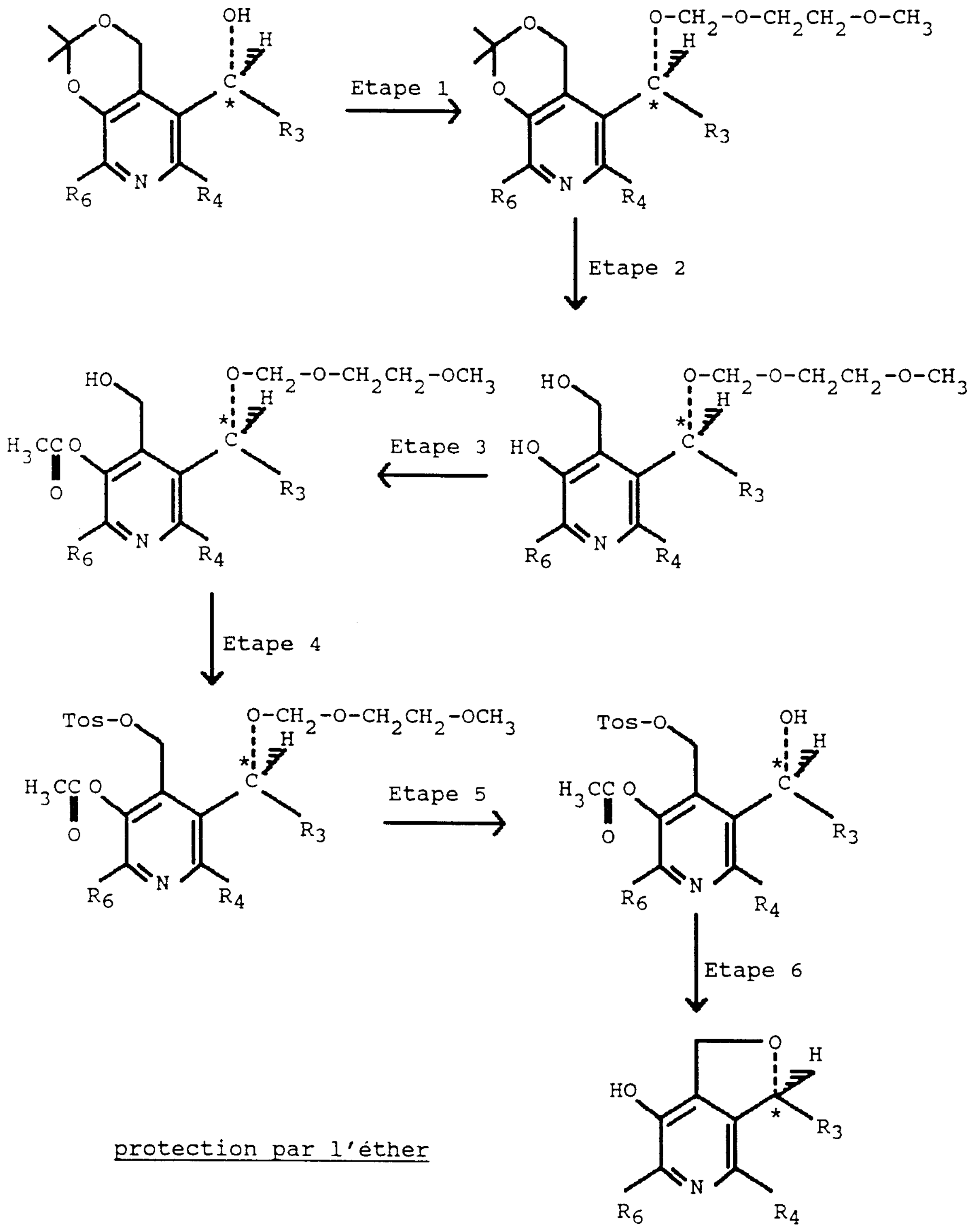


protection par l'éther

SCHEMA 3

dérivés (-) de la furo[3,4-c]pyridine

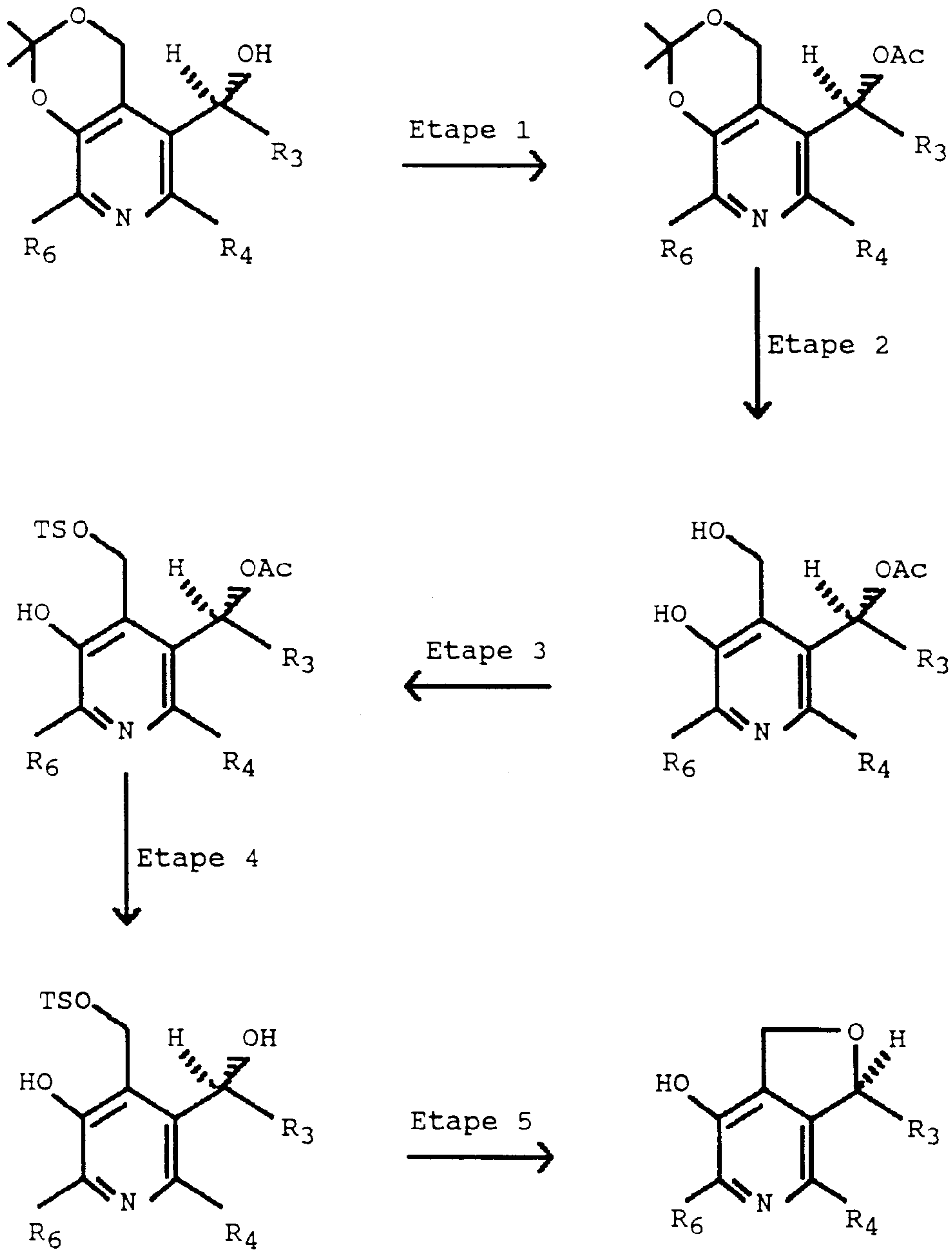


protection par l'ester

SCHEMA 4

dérivés (+) de la furo[3,4-c]pyridine

La méthode s'applique aisément aux différents substituants en position $R_3$ et l'invention sera mieux comprise grâce à la description de la synthèse de la forme (+) du dérivé de la furopyridine dans laquelle $R_4$ représente H, $R_6$ représente $CH_3$ et $R_3$ représente le p-chlorophényle. La description est donnée par les deux voies.

L'alcool secondaire de départ peut être obtenu comme décrit dans les précédents brevets espagnol No. 509 449, grec No. 75 905 et italien No. 1 157 252.

PREPARATION DE :

(+) ou (-) (chlorophényl-4)-3 dihydro-1,3 hydroxy-7 méthyl-6 furo [3,4-c] pyridine.

EXEMPLE SELON LA VOIE 1

Etape 1

triméthyl-2,2,8 (chloro-4-α-chlorobenzyl)-5 pyrido-[4,3-e]-dioxane-1,3.

Dans une fiole de 5 ml, on verse 88 mg (0,275 mmole) de (+) triméthyl-2,2,8 (chloro-4-α-hydroxybenzyl)-5 pyrido-[4,3-e]-dioxane-1,3, dissous dans 1 ml de chlorure de méthylène et la fiole est fermée hermétiquement sous atmosphère d'azote. 22 µl (0,27 mmole) de pyridine anhydre sont injectés via une seringue en verre. 36 µl (0,54 mmole) de chlorure de thiényle sont ensuite ajoutés goutte à goutte. Après disparition (2 heures plus tard) de la matière première (contrôlée par TLC), le solvant est éliminé par évaporation sous pression réduite.

Le résidu est ensuite dissout dans du chlorure de méthylène (50 ml), le chlorhydrate de pyridinium est filtré et la solution est transférée dans un entonoir séparateur. La fraction de chlorure de méthylène est lavée deux fois avec 10 ml de HCl 2N, la couche organique est séchée sur $Na_2SO_4$, filtrée et évaporée à sec sur roto vac (90 mg). Rendement : 96,5 %.

**Etape 2**

(+)(chlorophényl-4)-3 dihydro-1,3 hydroxy-7 méthyl-6 furo [3,4-c]pyridine

90 mg de (-)triméthyl-2,2,8 (chloro-4-α-chlorobenzyl) -5 pyrido-[4,3-e] dioxane-1,3 sont dissous dans 1 ml de TFA/$H_2O$ (9:1) et le taux de clivage de l'acétal est contrôlé par HPLC. Après 18 heures, le solvant est extrait sous circulation d'azote et concentré sous pression réduite. Le résidu est dissout dans l'ammoniaque méthanolique et séché sous circulation d'azote.

Le résidu brut est ensuite dissout dans le méthanol (1 ml) et chauffé pendant 30 minutes. Après refroidissement et filtration, un solide cristallise, dont l'analyse élémentaire montre qu'il correspond parfaitement à la formule du composé recherché. Rendement : 66 %, m = 52 mg.

Si on utilise au départ (-)triméthyl-2,2,8 (chloro-4-α-hydroxybenzyl)-5 pyrido-[4,3-e]-dioxane-1,3, le dérivé (-) correspondant est obtenu avec un rendement similaire.

**EXEMPLE SELON LA VOIE 2 (Schéma 3)**

**Etape 1**

**(+)triméthyl-2,2,8 [chloro-4-α-(méthoxy éthoxy-2 méthoxy) benzyl]-5 pyrido [4,3-e]-dioxane-1,3**

Dans un réacteur d'un litre, on prépare une solution de 3,8 g (11,8 mmoles) de (+)triméthyl-2,2,8 (chloro-4-α-hydroxybenzyl)-5 pyrido-[4,3-e]-dioxane-1,3 (90 % (+), 10 % (-)) dans 24 ml de tétrahydrofurane anhydre.

0,44 g (14,8 mmoles) d'hydrure de sodium à 80 % en dispersion dans l'huile sont ensuite ajoutés à une température de 0 °C. Le mélange réactionnel est agité à 20 °C pendant 4 heures puis refroidi à 0 °C. 2,2 g (17,8 mmoles) de chlorure de méthoxy éthoxy-2 méthyle sont ajoutés en 10 minutes. L'agitation est maintenue pendant 17 heures à 20 °C.

Une solution saturée de bicarbonate de sodium est ajoutée et la phase organique obtenue après décantation est séchée sur sulfate de sodium.

Après filtration et élimination du solvant, on obtient une huile orange qui, après purification par HPLC préparatoire, donne 2,4 g d'une huile limpide de couleur jaune avec un rendement de 49,5 %.

L'identité et la structure de ce composé sont confirmées par RMN$^1$H et microanalyse.

La composition énantiomérique donnée par HPLC est de 90 % (+), 10 % (-).

**Etape 2**

**(+)méthyl-2 hydroxy-3 hydroxyméthyl-4 [chloro-4-α-méthoxy éthoxy-2 méthoxy)-benzyl]-5 pyridine**

2,4 g (5,8 mmoles) de (+)triméthyl-2,2,8 [chloro-4-α-(méthoxyéthoxy-2 méthoxy)-benzyl]-5 pyrido-[4,3-e]-dioxane-1,3 sont versés dans un réacteur de 50 ml et traités par 20 ml d'une solution acide trifluoroacétique/eau 1:1 à température ambiante.

Le mélange réactionnel est chauffé à 40 ° C pendant 4 heures. Le solvant est évaporé sous vide. L'huile obtenue est reprise avec 25 ml d'ammoniaque méthanolique. Le solvant est à nouveau éliminé par évaporation sous vide et le résidu est traité par 100 ml de dichlorométhane.

L'insoluble est éliminé par filtration. Le filtrat est concentré sous vide pour obtenir 1,75 g d'une huile marron. Rendement : 81 %.

Le produit est purifié par HPLC ; on obtient 1,12 g d'une poudre blanche (rendement global 52 %).

L'identité et la structure de ce composé sont confirmées par RMN$^1$Het microanalyse.

La composition énantiomérique ne peut pas être obtenue par HPLC.

**Etape 3**

**(+)méthyl-2 acétyloxy-3 hydroxyméthyl-4 [chloro-4-α-méthoxy éthoxy-2 méthoxy)-benzyl]-5 pyridine**

Dans un réacteur de 50 ml, on dissout 0,7 g (1,9 mmoles) de (+)méthyl-2 hydroxy-3 hydroxyméthyl-4 [chloro-4-α-méthoxy éthoxy-2 méthoxy)-benzyl]-5 pyridine dans 15 ml de toluene anhydre. On ajoute, sous agitation, 0,3 ml (2,46 mmoles) de N,N-diméthylaniline, suivi par 0,5 ml (2,46 mmoles) d'anhydride acétique.

Le mélange réactionnel est maintenu à 40 ° C pendant 6 heures, puis refroidi et lavé trois fois avec 50 ml d'une solution aqueuse saturée de chlorure de sodium. La phase organique est séchée sur sulfate de sodium, filtrée et le solvant est éliminé sous vide.

On obtient 0,8 g d'une huile marron qui, purifiée par HPLC, donne 0,39 g d'une huile jaune ; rendement 50 %.

L'identité et la structure de ce composé sont confirmées par RMN$^1$H et microanalyse.

La composition énantiomérique ne peut pas être obtenue par HPLC.

**Etape 4**

**(+)méthyl-2 acétyloxy-3 tosyloxy méthyl-4 [(chloro-4-α-méthoxy éthoxy-2 méthoxy)-benzyl]-5 pyridine**

Dans un réacteur de 50 ml on verse 0,3 g (1,5 mmole) de chlorure de tosyle dans 10 ml d'acétone. On ajoute, à température ambiante, dans 10 ml d'acétone, 0,3 g (0,7 mmoles) d'une solution de (+)méthyl-2 acétyloxy-3 hydroxyméthyl-4 [(chloro-4-α-méthoxy éthoxy-2 méthoxy)-benzyl]-5 pyridine.

On ajoute ensuite 0,14 g (1 mmole) de carbonate de potassium. Le mélange réactionnel est porté au reflux pendant 4 heures et filtré.

Le filtrat est concentré sous vide. 0,76 g d'une huile marron sont purifiés par HPLC. On obtient 0,3 g d'une huile épaisse de couleur jaune avec un rendement de 75 %.

L'identité et la structure de ce composé sont confirmés par RMN$^1$H et microanalyse.

La composition énantiomérique obtenue par HPLC est de 90 % (+), 10 % (-).

**Etape 5**

**(+)méthyl-2 acétyloxy-3 tosyloxy méthyl-4 [chloro-4-α-hydroxy benzyl]-5 pyridine**

Dans un réacteur de 0,1 litre, sous circulation d'azote, on prépare, à température ambiante, une solution de 0,3 g (0,53 mmoles) de (+)méthyl-2 acétyloxy-3 méthyl tosyloxy-4 [(chloro-4-α-méthoxy éthoxy-2 méthoxy)-benzyl]-5 pyridine dans 20 ml de $CH_3CN$.

On ajoute ensuite 0,8 g (5,3 mmoles) d'iodure de sodium et 0,7 ml (5,3 mmoles) de chlorotriméthylsilane. La suspension orange résultante est agitée à température ambiante pendant 1 heure. L'hydrolyse du mélange réactionnel est réalisée à température ambiante, par 20 ml de méthanol. Les solvants sont éliminés par évaporation et l'huile restante est reprise par 50 ml d'éther éthylique, lavée deux fois avec 50

ml d'une solution de thiosulfate de sodium et deux fois avec une solution aqueuse saturée de chlorure de sodium. La phase organique est séchée sur sulfate de sodium.

Après filtration et élimination du solvant, on obtient une huile épaisse de couleur marron qui, après purification par HPLC donne 0,157 g d'une poudre blanche, avec un rendement de 63 %.

L'identité et la structure de ce composé sont confirmées par RMN$^1$H et microanalyse.

La composition énantiomérique donnée par HPLC est de 90 % (+), 10 % (-).

**Etape 6**

**(+)(chlorophényl-4)-3 dihydro-1,3 hydroxy-7 méthyl-6 furo [3,4-c]pyridine**

Dans une fiole de 10 ml, sous atmosphère d'azote, on prépare la solution de 100 mg (0,2 mmole) de (+)méthyl-2 acétyloxy-3 tosyloxy méthyl-4 [chxoro-4-α-hydroxybenzyl] -5 pyridine dans 1 ml de DMF.

On ajoute 12 mg (0,4 mmole) d'hydrure de sodium (80 % en dispersion dans l'huile) sous agitation à 20 °C. Le mélange réactionnel est agité à température ambiante pendant 1 heure, puis dilué avec 5 ml d'eau et acidifié par 1 ml de HCl 6N.

Le produit est récupéré par filtration et purifié par HPLC. On obtient 44 mg d'une poudre blanche avec un rendement de 50 %.

L'identité et la structure de ce composé ont été confirmées par RMN$^1$H et microanalyse.

La composition énantiomérique donnée par HPLC est de 80 % (+), 20 % (-).

Cette valeur représente une racémisation globale d'approximativemènt 10 %, basée sur le (+)triméthyl-2,2,8 (chloro-4-α-hydroxybenzyl)-5 pyrido-[4,3-e]-dioxane-1,3 de départ.

En partant du dérivé (-) correspondant, dans l'étape 1, on obtient le dérivé final (-) correspondant, avec un rendement légèrement inférieur.

**EXEMPLE SELON LA VOIE 2 (Schéma 4)**

**Etape 1**

**triméthyl-2,2,8 (chloro-4-α-acetoxybenzyl)-5 pyrido-[4,3-e]-dioxane-1,3**

210 mg (0,66 mmoles) de (+)triméthyl-2,2,8 (chloro-4-α-hydroxybenzyl)-5 pyrido-[4,3-e]-dioxane-1,3 sont versés dans une fiole de 2 ml et dissous dans 500 µl de pyridine et 110 µl d'anhydre acétique (1,16 mmole). Le mélange réactionnel est agité pendant 18 heures, puis versé dans 30 ml d'une solution saturée de $NaHCO_3$. Le mélange réactionnel est maintenu sous agitation à température ambiante pendant 1 heure, puis repris par 3 x 30 ml de $CH_2Cl_2$. Les fractions de $CH_2Cl_2$ rassemblées sont lavées avec 3 x 20 ml de HCl 2H.

La phase organique est séchée sur $Na_2SO_4$, filtrée et le filtrat est évaporé à sec.

On obtient 24 g d'un solide blanc, avec un rendement de 100 %.

Il a été démontré que ce composé était conforme par TLC (silice) et chimiquement pur (99,1 %) par HPLC. La pureté optique, confirmée par HPLC, est de 6,2 % (-) et 93,8 % (+).

**Etape 2**

**(+)méthyl-2 hydroxy-3 hydroxyméthyl-4 [chloro-4-α-acetoxybenzyl]-5 pyridine**

0,24 g (0,66 mmoles) de (+)triméthyl-2,2,8 (chloro-4-α-acétoxybenzyl)-5 pyrido-[4,3-e]-dioxane-1,3 sont versés dans un récipient de 10 ml à fond rond et dissous dans 3,3 ml de TFA/$H_2O$ (10:1). Le mélange réactionnel est maintenu sous agitation à température ambiante pendant 1,5 heure. Le solvant est ensuite evacué au rotavator. On ajoute 1 ml d'ammoniac en solution méthanolique au produit brut (l'excédent de TFA est neutralisé), qui est ensuite évaporé à sec sous vide.

Par HPLC on a montré que le produit brut contenait 90 % de l'acétate-diol recherché ainsi que 4,4 % de la matière de départ n'ayant pas réagi.

**Etapes 3 et 4**

**(+)méthyl-2 hydroxy-3 tosyloxyméthyl-4 [chloro-4-α-hydroxybenzyl]-5 pyridine**

0,24 g (0,66 mmoles) de (+)méthyl-2 hydroxy-3 hydroxyméthyle-4 [chloro-4-α-acétoxybenzyl]-5 pyridine sont versés dans un récipient de 25 ml à fond rond et dissous dans 10 ml de chlorure de méthylène. On ajoute alors 190 mg (1 mmole) de chlorure p-toluène sulphonyl et 75 mg (1 mmole) de pyridine. Le mélange réactionnel est agité à température ambiante pendant 18 heures ; le solvant est ensuite évacué sous pression réduite. On ajoute 5 ml d'ammoniac en solution méthanolique et le mélange est agité à température ambiante pendant 2 heures. Le solvant est évacué sous vide, le produit obtenu dissout à nouveau dans 1 ml de méthanol et purifié par TLC (silice).

On obtient 170 mg du composé recherché sous la forme d'un solide blanc légèrement teinté.

**Etape 5**

**(+)(chlorophényl-4)-3 dihydro-1,3 hydroxy-7 méthyl-6-furo-[3,4-c]pyridine**

62 mg (0,144 mmoles) de (+)méthyl-2 hydroxy-3 tosyloxyméthyl-4 [chloro-4-α-hydroxybenzyl]-5 pyridine sont versés dans une fiole de 2 ml et dissous dans 500 µl de HMPT. On ajoute alors 14 mg (0,3 mmoles) de NaH (50 % en dispersion dans l'huile) et le mélange est agité à température ambiante pendant 1 heure. Le mélange réactionnel est ensuite dilué avec 2,5 ml de $H_2O$ et acidifié par 2 gouttes de HCl 6N. Le précipité solide du composé recherché qui se forme est séparé par filtration.

Le solide brut est dissout dans du méthanol, placé sur une plaque de silice TLC de 1000 µ et élué par $CH_2Cl_2$/MeOH (7:1). La bande UV principale, éluée en même temps qu'une tâche authentique de (+)-(chlorophényl-4)-3 dihydro-1,3 hydroxy-7 méthyl-6-furo[3,4-c]pyridine, est coupée et le composé est récupéré par lavage de la silice au méthanol.

Le filtrat de méthanol est évaporé, ce qui donne 27 mg d'un solide blanc.

Ce composé s'est avéré être le composé recherché en le co-éluant avec (+)(chlorophényl-4)-3 dihydro-1,3 hydroxy-7 méthyl-6-furo-[3,4-c]pyridine authentique sur TLC et HPLC.

La pureté optique de l'échantillon, donnée par HPLC, est de 14,6 % (-) et 85,4 % (+).

Cette valeur représente une racémisation globale de 7,5 %, basée sur le (+)triméthyl-2,2,8 (chloro-4-α-hydroxybenzyl)-5 pyrido-[4,3-e]-dioxane-1,3 de départ.

On a préparé la (+)(chlorophényl-4)-3 dihydro-1,3 hydroxy-7 méthyl-6-furo-[3,4-c]pyridine à partir du (+)triméthyl-2,2,8 (chloro-4-α-hydroxybenzyl)-5 pyrido-[3,4-e]-dioxane-1,3 avec un rendement global d'approximativement 45 % pour la séquence de 5 étapes.

La préparation de la (-)(chlorophényl-4)-3 dihydro-1,3 hydroxy-7 méthyl-6-furo-[3,4-c]pyridine à partir du (-)triméthyl-2,2,8 (chloro-4-α-hydroxybenzyl)-5 pyrido-[3,4-e]-dioxane-1,3 selon la même voie a été obtenue avec un rendement global légèrement supérieur (48 %).

Les composés obtenus selon la présente invention présentent un intérêt dans les divers domaines pharmaceutiques décrits dans les brevets précédemment mentionnés en page 2.

En conséquence, cette invention concerne également des compositions pharmaceutiques, dont l'ingrédient actif est un énantiomère ou des mélanges d'énantiomères dans lesquels l'un des énantiomères est largement prédominant.

Les types d'administration appropriés sont décrits dans les brevets mentionnés précédemment en page 2, mais le dosage est plus faible que dans lesdits brevets, ce qui s'explique par l'activité renforcée de l'énantiomère choisi ou des mélanges d'énantiomères dans lesquels l'énantiomère en question est largement prédominant.

Ceci est démontré, par exemple, par l'action diurétique comparée sur le rat normal WKY des deux formes énantiomères dont la préparation a été décrite plus haut avec le racémique correspondant. L'expérimentation a été conduite sur des lots de 10 rats aux doses de 10 ou de 20 mg/kg, per os, chaque animal recevant 2,5 ml, par 100 g de poids, de sérum physiologique, soit pur, pour le lot de témoins, soit additionné de la quantité appropriée du produit à tester, pour les animaux traités par le racémique ou chacun des énantiomères.

Les résultats ont été reportés dans le tableau ci-après. On peut noter que l'isomère (+) à 10 mg/kg est au moins aussi actif que le racémique à 20 mg/kg et qu'à ces doses, il est 58 % meilleur pour le taux d'élimination $Na^+/K^+$. De plus, il y a un plafonnement de la relation dose/effet et chez le rat normotendu, la dose optimale se situe vers 6 mg/kg per os.

Une expérimentation comparable effectuée sur le rat génétiquement hypertendu SHR-SP à la dose de 3 mg/kg, per os, tant pour les énantiomères que pour le racémique, donne des volumes urinés à 6 heures, supérieurs de 10 % pour le dérivé (+) par rapport au racémique.

| Produits | | Témoins | Pacémique 20 mg/Kg PO | Isomère (+) 10 mg/Kg PO | Isomère (+) 20 mg/Kg PO | Isomère (-) 10 mg/Kg PO | Isomère (-) 20 mg/Kg PO |
|---|---|---|---|---|---|---|---|
| Volume urine | ml/6 h | 1,71 ± 0,180 — | 4,71 ± 0,314 *** | 4,76 ± 0,338 *** | 4,44 ± 0,467 *** | 2,48 ± 0,245 NS | 2,72 ± 0,174 * |
| | % Var. | | 175 | 178 | 160 | 45 | 59 |
| Acide urique | $10^{-3}$ mmol/ 6 h | 3,15 ± 0,269 — | 2,35 ± 0,142 *** | 2,29 ± 0,103 *** | 1,94 ± 0,138 *** | 2,49 ± 0,101 ** | 2,5 ± 0,188 ** |
| | % Var. | | - 25 | - 27 | - 38 | - 21 | - 21 |
| $Cl^-$ | $10^{-3}$ mEq/ 6 h | 356,6 ± 31,56 — | 896,5 ± 32,67 *** | 869,2 ± 33,91 *** | 879,9 ± 41,70 *** | 467,5 ± 26,26 * | 517 ± 20,96 *** |
| | % Var. | | 151 | 144 | 147 | 31 | 45 |
| $K^+$ | $10^{-3}$ mEq/ 6 h | 124,2 ± 12 — | 195,6 ± 11,56 *** | 169,4 ± 15,65 ** | 180,6 ± 13,58 ** | 156,7 ± 11,6 NS | 159,4 ± 12,42 NS |
| | % Var. | | 57 | 36 | 45 | 26 | 28 |
| $Na^+$ | $10^{-3}$ mEq/ 6 h | 282,1 ± 24,57 — | 780,5 ± 33,01 *** | 767,7 ± 31,22 *** | 749,1 ± 42,73 *** | 387 ± 27,37 * | 422,5 ± 20,56 ** |
| | % Var. | | 177 | 172 | 166 | 37 | 50 |
| $\frac{Na^+}{K^+}$ | Rapport | 2,25 ± 0,222 — | 4,08 ± 0,147 *** | 5,12 ± 0,525 *** | 4,34 ± 0,268 *** | 2,55 ± 0,163 NS | 2,83 ± 0,200 NS |
| | % Var. | | 81 | 128 | 93 | 13 | 26 |

## PRESENTATION

Le mode préféré d'administration comprend des comprimés et des gélules. Pour les comprimés, chaque unité de dosage renferme de 5 à 100 mg ou, de préférence, 10 ou 25 mg de principe actif associé

avec un excipient approprié comme, par exemple, l'amidon.

## POSOLOGIE

En thérapeutique humaine, les doses à utiliser sont de 50 à 150 mg/jour pour au moins une semaine et, de préférence, pour des durées plus longues.

**Revendications**

**Revendications pour les Etats contractants suivants : ES, GR**

1. Procédé stéréospécifique pour la préparation des énantiomères (+) de la furo [3,4-c] pyridine substituée en position 3, répondant à la formule générale I:

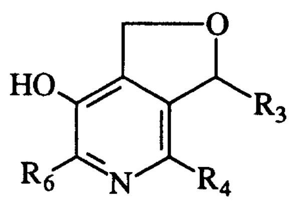

I

dans laquelle
$R_3$ représente un radical hydrocarboné, saturé ou non saturé, en chaîne droite ou ramifiée, comportant de 1 à 5 atomes de carbone, un groupement hétérocyclique ayant jusqu'à 6 atomes de carbone dans le cycle, un groupement phényle ou cyclohexyle, un groupement alcoylphényle ou alcénylphényle, chacun de ces groupements étant éventuellement substitué par un ou plusieurs atomes de chlore, de brome ou de fluore, un ou plusieurs groupements trifluorométhyle, alcoyle ayant de 1 à 5 atomes de carbone, alcoxy ayant de 1 à 5 atomes de carbone, alcoylthio ayant de 1 à 5 atomes de carbone, dialcoylamino dans lequel chacun des groupements alcoyle comprend de 1 à 5 atomes de carbone, dialcoylaminoalcoxy dans lesquels chacun des deux groupements alcoyle et le groupement alcoxy contient de 1 à 5 atomes de carbone ou un groupement $\alpha$- ou $\beta$-alcoxy-N-pyrrolidinyle dans lequel l'alcoxy contient de 1 à 5 atomes de carbone;
$R_4$ représente un atome d'hydrogène ou d'halogène et
$R_6$ représente un groupememt alcoyle ou alcényle en chaîne droite ou ramifiée ayant jusqu'à 6 atomes de carbone, éventuellement substitué par un radical hydroxy, cyano, amino ou un radical amino substitué ou par un radical alcoyle ou alcényle ayant jusqu'à 4 atomes de carbone,
comprenant les étapes suivantes :

(a) Oxydation, par un agent oxydant quelconque, d'un dérivé racémique de la pyridine répondant à la formule générale II

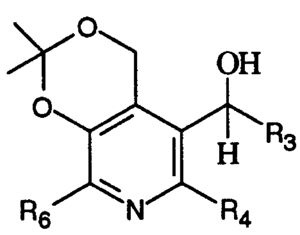

II

dans laquelle $R_3$, $R_4$ et $R_6$ sont tels que définis ci-dessus, par un agent oxydant quelconque ;

(b) Réduction de la cétone obtenue répondant à la formule générale III

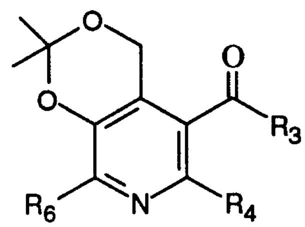

III

dans laquelle $R_3$, $R_4$ et $R_6$ sont tels que définis ci-dessus, caractérisée en ce que l'on emploie un agent réducteur chiral ou un catalyseur approprié pour une hydrogénation asymétrique, de façon à obtenir le composé suivant :

III + agent réducteur stéréospécifique

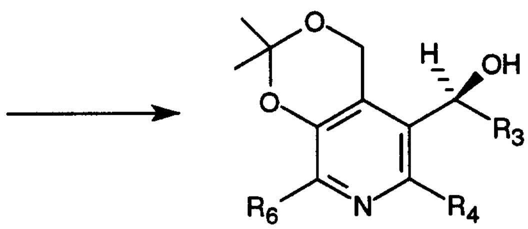

IV (+)

(c) verrouillage ou blocage stéréospécifique du groupe OH de l'alcool énantiomère (+);
(d) ouverture du cycle acétonide par des acides protiques avec libération concomitante des groupes $CH_2OH$ et OH sur le cycle pyridine ;
(e) cyclisation du composé obtenu et, si nécessaire, déprotection du groupement phénoxy.

**2.** Procédé selon la revendication 1, dans lequel l'agent de verrouillage est un atome d'halogène substitué au centre carbinol de l'alcool énantiomère.

**3.** Procédé selon la revendication 2, dans lequel l'atome d'halogène est un atome de chlore.

**4.** Procédé selon la revendication 3, dans lequel l'agent de chloration est choisi parmi la triphénylphosphine avec du tétrachlorure de carbone ou $SO_2Cl_2$ avec du chlorure de méthylène.

**5.** Procédé selon la revendication 1, dans lequel l'agent de blocage est constitué par le groupement éther, le dit groupement éther étant obtenu par réaction avec un composé de formule RX dans lequel X représente un atome d'halogène et R un groupement aryle ou aralcoyle ou alcoyle ou alcoxy-alcoxy-alcoyle, tous les groupements alcoyle ayant jusqu'à 5 atomes de carbone, ou un groupement tertio butyle silyle.

**6.** Procédé selon la revendication 5, dans lequel R représente le radical méthoxy éthoxy-2 méthyle.

**7.** Procédé selon la revendication 1, dans lequel l'agent de blocage est constitué par le groupement ester, le dit groupement ester étant obtenu par réaction avec un composé de formule RCOX ou $(RCO)_2O$ dans lequel X représente un atome d'halogène et R un groupement alcoyle inférieur ayant jusqu'à 5 atomes de carbone.

**8.** Procédé selon la revendication 7, dans lequel R représente le radical méthyle.

**Revendications pour l'Etat contractant suivant : IT**

**1.** Procédé stéréospécifique pour la préparation des énantiomères (+) de la furo [3,4-c] pyridine substituée en position 3, répondant à la formule générale I:

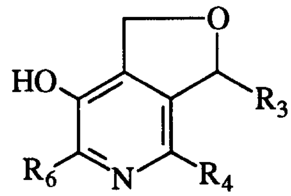

I

dans laquelle

$R_3$ représente un radical hydrocarboné, saturé ou non saturé, en chaîne droite ou ramifiée, comportant de 1 à 5 atomes de carbone, un groupement hétérocyclique ayant jusqu'à 6 atomes de carbone dans le cycle, un groupement phényle ou cyclohexyle, un groupement alcoylphényle ou alcénylphényle, chacun de ces groupements étant éventuellement substitué par un ou plusieurs atomes de chlore, de brome ou de fluore, un ou plusieurs groupements trifluorométhyle, alcoyle ayant de 1 à 5 atomes de carbone, alcoxy ayant de 1 à 5 atomes de carbone, alcoylthio ayant de 1 à 5 atomes de carbone, dialcoylamino dans lequel chacun des groupements alcoyle comprend de 1 à 5 atomes de carbone, dialcoylaminoalcoxy dans lesquels chacun des deux groupements alcoyle et le groupement alcoxy contient de 1 à 5 atomes de carbone ou un groupement $\alpha$- ou $\beta$-alcoxy-N-pyrrolidinyle dans lequel l'alcoxy contient de 1 à 5 atomes de carbone;

$R_4$ représente un atome d'hydrogène ou d'halogène et

$R_6$ représente un groupememt alcoyle ou alcényle en chaîne droite ou ramifiée ayant jusqu'à 6 atomes de carbone, éventuellement substitué par un radical hydroxy, cyano, amino ou un radical amino substitué ou par un radical alcoyle ou alcényle ayant jusqu'à 4 atomes de carbone,

comprenant les étapes suivantes :

(a) Oxydation, par un agent oxydant quelconque, d'un dérivé racémique de la pyridine répondant à la formule générale II

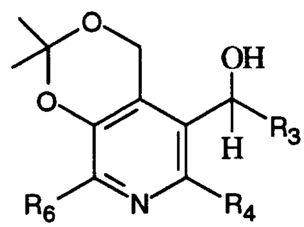

II

dans laquelle $R_3$, $R_4$ et $R_6$ sont tels que définis ci-dessus, par un agent oxydant quelconque ;

(b) Réduction de la cétone obtenue répondant à la formule générale III

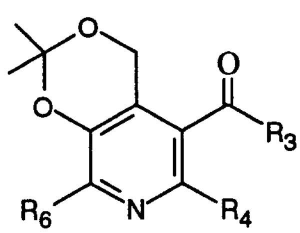

III

dans laquelle $R_3$, $R_4$ et $R_6$ sont tels que définis ci-dessus, caractérisée en ce que l'on emploie un agent réducteur chiral ou un catalyseur approprié pour une hydrogénation asymétrique, de façon à obtenir le composé suivant :

III + agent réducteur stéréospécifique

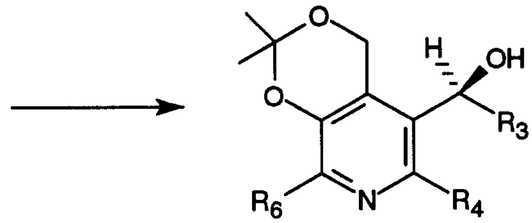

IV (+)

(c) verrouillage ou blocage stéréospécifique du groupe OH de l'alcool énantiomère (+);
(d) ouverture du cycle acétonide par des acides protiques avec libération concomitante des groupes $CH_2OH$ et OH sur le cycle pyridine ;
(e) cyclisation du composé obtenu et, si nécessaire, déprotection du groupement phénoxy.

2. Procédé selon la revendication 1, dans lequel l'agent de verrouillage est un atome d'halogène substitué au centre carbinol de l'alcool énantiomère.

3. Procédé selon la revendication 2, dans lequel l'atome d'halogène est un atome de chlore.

4. Procédé selon la revendication 3, dans lequel l'agent de chloration est choisi parmi la triphénylphosphine avec du tétrachlorure de carbone ou $SO_2Cl_2$ avec du chlorure de méthylène.

5. Procédé selon la revendication 1, dans lequel l'agent de blocage est constitué par le groupement éther, le dit groupement éther étant obtenu par réaction avec un composé de formule RX dans lequel X représente un atome d'halogène et R un groupement aryle ou aralcoyle ou alcoyle ou alcoxy-alcoxy-alcoyle, tous les groupements alcoyle ayant jusqu'à 5 atomes de carbone, ou un groupement tertio butyle silyle.

6. Procédé selon la revendication 5, dans lequel R représente le radical méthoxy éthoxy-2 méthyle.

7. Procédé selon la revendication 1, dans lequel l'agent de blocage est constitué par le groupement ester, le dit groupement ester étant obtenu par réaction avec un composé de formule RCOX ou $(RCO)_2O$ dans lequel X représente un atome d'halogène et R un groupement alcoyle inférieur ayant jusqu'à 5 atomes de carbone.

8. Procédé selon la revendication 7, dans lequel R représente le radical méthyle.

9. Enantiomères (+) de la furo [3,4-c] pyridine substituée en position 3, tels qu'obtenus par le procédé selon l'une des revendications 1 à 8, et répondant à la formule I

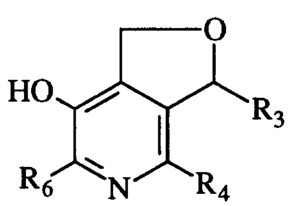

I

dans laquelle
$R_3$ représente un radical hydrocarboné, saturé ou non saturé, en chaîne droite ou ramifiée, comportant de 1 à 5 atomes de carbone, un groupement hétérocyclique ayant jusqu'à 6 atomes de carbone dans le cycle, un groupement phényle ou cyclohexyle, un groupement alcoylphényle ou alcénylphényle, chacun de ces groupements étant éventuellement substitué par un ou plusieurs atomes de chlore, de brome ou de fluor, un ou plusieurs groupements trifluorométhyle, alcoyle ayant de 1 à 5 atomes de carbone, alcoxy ayant de 1 à 5 atomes de carbone, alcoylthio ayant de 1 à 5 atomes de carbone, dialcoylamino dans lequel chacun des groupements alcoyle comprend de 1 à 5 atomes de carbone, dialcoylaminoalcoxy dans lesquels chacun des deux groupements alcoyle et le groupement alcoxy

contient de 1 à 5 atomes de carbone ou un groupement α- ou β-alcoxy-N-pyrrolidinyle dans lequel l'alcoxy contient de 1 à 5 atomes de carbone;
$R_4$ représente un atome d'hydrogène ou d'halogène et
$R_6$ représente un groupement alcoyle ou alcényle en chaîne droite ou ramifiée ayant jusqu'à 6 atomes de carbone, éventuellement substitué par un radical hydroxy, cyano, amino ou un radical amino substitué ou par un radical alcoyle ou alcényle ayant jusqu'à 4 atomes de carbone,
ou mélanges des énantiomères (+) et (-) dans lesquels l'énantiomère (+) est largement prédominant.

**10.** Enantiomère (+) de la furo [3,4-c] pyridine substituée en position 3, selon la revendication 9, dans lesquels $R_3$ représente p-chlorophényle, $R_4$ représente H et $R_6$ représente $CH_3$, ou mélange des énantiomères (+) et (-) de ce corps dans lequel l'énantiomère (+) est largement prédominant.

**11.** Compositions thérapeutiques dont l'ingrédient actif est une quantité suffisante du composé selon la revendication 9, associé à tout diluant ou excipient approprié.

**12.** Compositions thérapeutiques selon la revendication 11, dans lesquelles, par unité de dosage, l'ingrédient actif est constitué par 5 à 100 mg de (+) (chlorophényl-4)-3 dihydro-1,3 hydroxy-7 méthyl-6 furo [3,4-c] pyridine, ou d'un mélange des énantiomères (+) et (-) de la (chlorophényl-4)-3 dihydro-1,3 hydroxy-7 méthyl-6 furo [3,4-c] pyridine, dans lequel l'énantiomère (+) est prédominant.

**Claims**
**Claims for the following Contracting States : ES, GR**

**1.** Stereospecific process for the preparation of (+) enantiomers of 3-substituted-furo [3,4-c] pyridine of the general formula I :

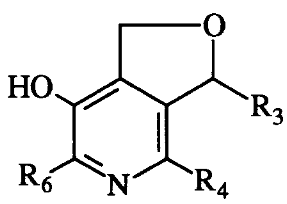

I

wherein
$R_3$ stands for a straight chain saturated or unsaturated hydrocarbon group having from 1 to 5 carbon atoms, a heterocyclic group having up to 6 ring atoms, a phenyl or cyclohexyl group, a phenylalkyl group or a phenylalkenyl group, each of said groups being optionally substituted by one or more chlorine bromine or fluorine atoms, trifluoromethyl groups, alkyl groups having from 1 to 5 carbon atoms, alkoxy groups having from 1 to 5 carbon atoms, alkylthio groups having from 1 to 5 carbon atoms, dialkylamino groups in which each alkyl group has from 1 to 5 carbon atoms, dialkylaminoalkoxy groups in which each of the two alkyl groups and the alkoxy group has from 1 to 5 carbon atoms or an α- or β-alkoxy-N-pyrrolidinyl group in which the alkoxy group has from 1 to 5 carbon atoms ;
$R_4$ stands for a hydrogen or a halogen atom and
$R_6$ stands for a straight chain or branched chain alkyl or alkenyl group having up to 6 carbon atoms, optionally substituted by a hydroxy, cyano, amino or substituted amino group or by an alkyl or alkenyl group having up to 4 carbon atoms,
comprising the steps of :

(a) Oxidising, by any usual oxidising agent, a racemic pyridine derivative of the general formula II

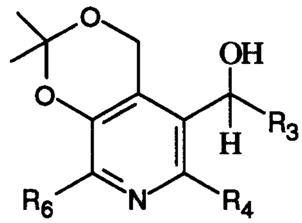

II

wherein $R_3$, $R_4$ and $R_6$ are as above defined, by any usual oxidising agent ;
(b) Reducing the resulting ketone of the general formula III

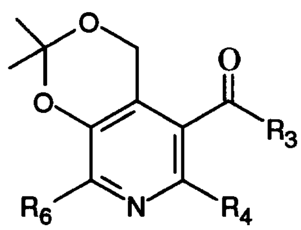

III

wherein $R_3$, $R_4$ and $R_6$ are as above defined, characterised in that, a chiral reducing agent or an appropriate catalyst for asymetrical hydrogenation is used, to obtain the following compound :

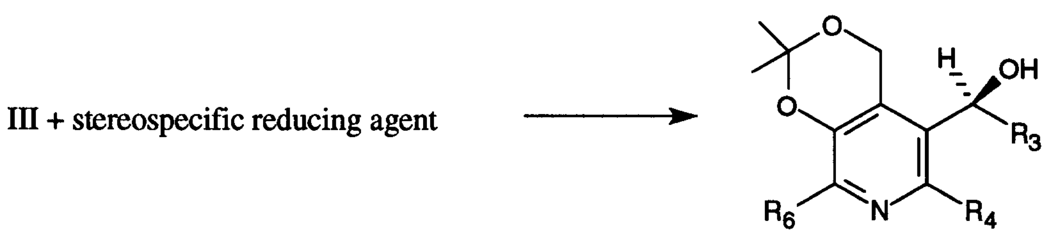


IV (+)

(c) Stereospecific locking or blocking of the OH group of the (+) enantiomer alcohol ;
(d) Opening of the acetonide ring by protic acids with concomitant liberation of the $CH_2OH$ and OH groups on the pyridine ring ;
(e) Cyclizing the resulting compound and, if necessary, deprotection of the phenoxy group.

**2.** Process according to claim 1 wherein the blocking agent is a halogen atom substituted at the carbinol centre of the enantiomer alcohol.

**3.** Process according to claim 2 wherein the halogen atom is a chlorine atom.

**4.** Process according to claim 3 wherein the chlorinating agent is selected of from triphenylphosphine with carbon tetrachloride and $SO_2Cl_2$ with methylene chloride.

**5.** Process according to claim 1 wherein the blocking agent is constituted by the ether group, said ether group being obtained by reacting a compound of formula RX, wherein X represents a halogen atom and R represents an aryl group, an aralkyl group in which the alkyl group has up to 5 carbon atoms, an alkyl group having up to 5 carbon atoms, an alkoxy-alkoxy-alkyl group in which each of the alkoxy groups and the alkyl group has up to 5 carbon atoms, or a *t*-butylsilyl group.

**6.** Process according to claim 5 wherein R represents a (2-methoxyethoxy)-methyl group.

7. Process according to claim 1 wherein the blocking agent is constituted by the ester group, said ester group being obtained by reacting a compound of formula RCOX or $(RCO)_2O$ wherein X represents a halogen atom and R represents a lower alkyl group having up to 5 carbon atoms.

8. Process according to claim 7 wherein R represents a methyl group.

**Claims for the following Contracting State : IT**

1. Stereospecific process for the preparation of (+) enantiomers of 3-substituted-furo [3,4-c] pyridine of the general formula I :

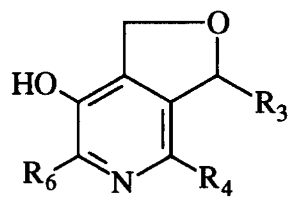

I

wherein
$R_3$ stands for a straight chain saturated or unsaturated hydrocarbon group having from 1 to 5 carbon atoms, a heterocyclic group having up to 6 ring atoms, a phenyl or cyclohexyl group, a phenylalkyl group or a phenylalkenyl group, each of said groups being optionally substituted by one or more chlorine bromine or fluorine atoms, trifluoromethyl groups, alkyl groups having from 1 to 5 carbon atoms, alkoxy groups having from 1 to 5 carbon atoms, alkylthio groups having from 1 to 5 carbon atoms, dialkylamino groups in which each alkyl group has from 1 to 5 carbon atoms, dialkylaminoalkoxy groups in which each of the two alkyl groups and the alkoxy group has from 1 to 5 carbon atoms or an α- or β-alkoxy-N-pyrrolidinyl group in which the alkoxy group has from 1 to 5 carbon atoms ;
$R_4$ stands for a hydrogen or a halogen atom and
$R_6$ stands for a straight chain or branched chain alkyl or alkenyl group having up to 6 carbon atoms, optionally substituted by a hydroxy, cyano, amino or substituted amino group or by an alkyl or alkenyl group having up to 4 carbon atoms,
comprising the steps of :

(a) Oxidising, by any usual oxidising agent, a racemic pyridine derivative of the general formula II

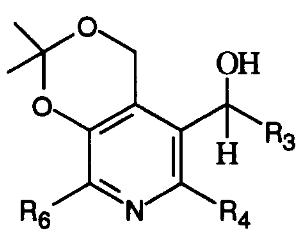

II

wherein $R_3$, $R_4$ and $R_6$ are as above defined, by any usual oxidising agent ;

(b) Reducing the resulting ketone of the general formula III

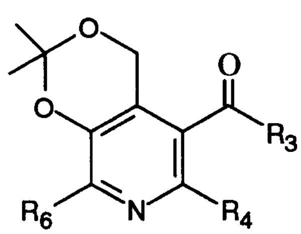

III

wherein $R_3$, $R_4$ and $R_6$ are as above defined, characterised in that, a chiral reducing agent or an appropriate catalyst for asymetrical hydrogenation is used, to obtain the following compound :

III + stereospecific reducing agent

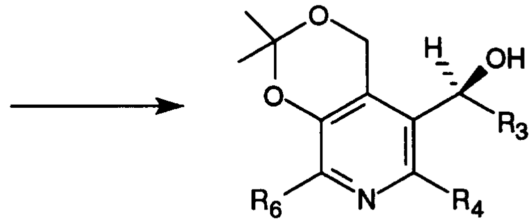

IV (+)

(c) Stereospecific locking or blocking of the OH group of the (+) enantiomer alcohol ;
(d) Opening of the acetonide ring by protic acids with concomitant liberation of the $CH_2OH$ and OH groups on the pyridine ring ;
(e) Cyclizing the resulting compound and, if necessary, deprotection of the phenoxy group.

**2.** Process according to claim 1 wherein the blocking agent is a halogen atom substituted at the carbinol centre of the enantiomer alcohol.

**3.** Process according to claim 2 wherein the halogen atom is a chlorine atom.

**4.** Process according to claim 3 wherein the chlorinating agent is selected of from triphenylphosphine with carbon tetrachloride and $SO_2Cl_2$ with methylene chloride.

**5.** Process according to claim 1 wherein the blocking agent is constituted by the ether group, said ether group being obtained by reacting a compound of formula RX, wherein X represents a halogen atom and R represents an aryl group, an aralkyl group in which the alkyl group has up to 5 carbon atoms, an alkyl group having up to 5 carbon atoms, an alkoxy-alkoxy-alkyl group in which each of the alkoxy groups and the alkyl group has up to 5 carbon atoms, or a *t*-butylsilyl group.

**6.** Process according to claim 5 wherein R represents a (2-methoxyethoxy)-methyl group.

**7.** Process according to claim 1 wherein the blocking agent is constituted by the ester group, said ester group being obtained by reacting a compound of formula RCOX or $(RCO)_2O$ wherein X represents a halogen atom and R represents a lower alkyl group having up to 5 carbon atoms.

**8.** Process according to claim 7 wherein R represents a methyl group.

**9.** (+) enantiomers of the 3-substituted-furo [3,4-c] pyridine, obtained according to the process of any of the preceding claims 1 to 8 and of the formula I

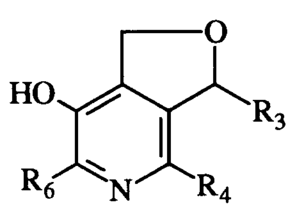

I

wherein
$R_3$ stands for a straight chain saturated or unsaturated hydrocarbon group having from 1 to 5 carbon atoms, a heterocyclic group having up to 6 ring atoms, a phenyl or cyclohexyl group, a phenylalkyl group or a phenylalkenyl group, each of said groups being optionally substituted by one or more chlorine bromine or fluorine atoms, trifluoromethyl groups, alkyl groups having from 1 to 5 carbon atoms, alkoxy groups having from 1 to 5 carbon atoms, alkylthio groups having from 1 to 5 carbon

atoms, dialkylamino groups in which each alkyl group has from 1 to 5 carbon atoms, dialkylaminoalkoxy groups in which each of the two alkyl groups and the alkoxy group has from 1 to 5 carbon atoms or an α- or β-alkoxy-N-pyrrolidinyl group in which the alkoxy group has from 1 to 5 carbon atoms ;
$R_4$ stands for a hydrogen or a halogen atom and
$R_6$ stands for a straight chain or branched chain alkyl or alkenyl group having up to 6 carbon atoms, optionally substituted by a hydroxy, cyano, amino or substituted amino group or by an alkyl or alkenyl group having up to 4 carbon atoms,
or mixtures of (+) and (-) enantiomers wherein the (+) enantiomer is largely predominant.

**10.** (+) enantiomer of the 3-substituted-furo [3,4-c] pyridine according to claim 9, wherein $R_3$ stands for p-chlorophenyl, $R_4$ stands for H and $R_6$ stands for $CH_3$, or mixture of (+) and (-) enantiomers of this compound wherein the (+) enantiomer is largely predominant.

**11.** Therapeutical compositions, in which the active ingredient is a sufficient amount of the compound according to claim 9, together with any appropriate diluent or carrier.

**12.** Therapeutical compositions according to claim 11, in which the active ingredient, per dosage unit, contains 5 to 100 mg of (+)-3-(4-chlorophenyl)-1,3-dihydro-7-hydroxy 6-methyl furo [3,4-c] pyridine, or a mixture of the (+) and (-) enantiomers of 3-(4-chlorophenyl)1,3-dihydro 7-hydroxy 6-methyl furo [3,4-c] pyridine wherein the (+) enantiomer is predominant.

**Patentansprüche**
**Patentansprüche für folgende Vertragsstaaten : ES, GR**

**1.** Stereospezifisches Verfahren zur Herstellung von (+) Enantiomeren von in 3-Stellung substituiertem Furo [3,4-c] Pyridins der allgemeinen Formel I :

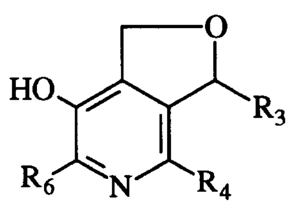

I

worin
$R_3$ einen gesättigten oder ungesättigten, geradkettigen oder verzweigten Kohlenwasserstoffrest mit 1 bis 5 Kohlenstoffatomen, eine heterocyclische Gruppe mit bis zu 6 Kohlenstoffatomen im Ring, eine Phenyl- oder Cyclohexylgruppe, eine Alkylphenyl- oder Alkenylphenylgruppe, jede dieser Gruppen gegebenenfalls substituiert mit einem oder mehreren Chlor-, Brom- oder Fluoratomen, einer oder mehreren Trifluormethylgruppen, Alkylgruppen mit 1 bis 5 Kohlenstoffatomen, Alkoxygruppen mit 1 bis 5 Kohlenstoffatomen, Alkylthiogruppen mit 1 bis 5 Kohlenstoffatomen, Dialkylaminogruppen, worin jede der Alkylgruppen 1 bis 5 Kohlenstoffatomen einschliesst, Dialkylaminoalkoxygruppen, worin jede der zwei Alkylgruppen und die Alkoxygruppe 1 bis 5 Kohlenstoffatomen enthält, oder einer α- oder β-Alkoxy-N-Pyrrolidinylgruppe, worin das Alkoxy 1 bis 5 Kohlenstoffatomen enthält, darstellt ;
$R_4$ ein Wasserstoff- oder Halogenatom darstellt und
$R_6$ eine verzweigte oder geradkettige Alkyl- oder Alkenylgruppe mit bis zu 6 Kohlenstoffatomen, gegebenenfalls substituierten durch einen Hydroxy-, Cyano-, Amino- oder substituierten Aminorest oder durch einen Alkyl- oder Alkenylrest mit bis zu 4 Kohlenstoffatomen, darstellt, welches die folgenden Schritte umfasst :

(a) Oxidation, mit einem beliebigen Oxidationsmittel, eines razemischen Derivats eines Pyridins der allgemeinen Formel II

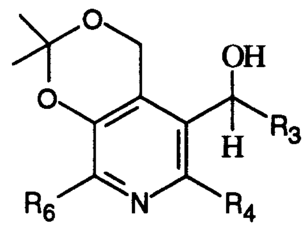

II

worin $R_3$, $R_4$ und $R_6$ wie vorstehend definiert sind, mit einem beliebigen Oxidationsmittel ;
(b) Reduktion des erhaltenen Ketons der allgemeinen Formel III

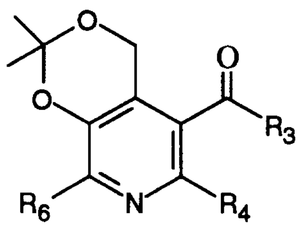

III

worin $R_3$, $R_4$ und $R_6$ wie vorstehend definiert sind, dadurch gekennzeichnet, daß ein beliebiges chirales Reduktionsmittel oder ein für eine asymmetrische Hydrierung geeigneten Katalysator verwendet werden, um die folgende Verbindung zu ergeben :

III + stereospezifisches Reduktionsmittel

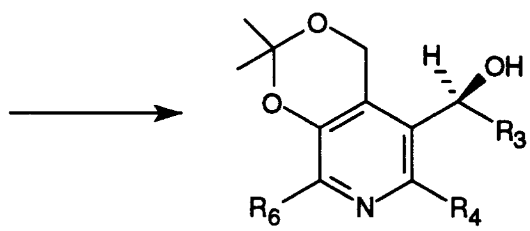

IV (+)

(c) Stereospezifisches Schützen oder Blockieren der OH-Gruppe des ( + ) enantiomeren Alkohols ;
(d) Öffnen des Acetonidrings mit protischen Säuren, unter gleichzeitiger Freisetzung der $CH_2OH$- und OH-Gruppe am Pyridinring ;
(e) Cyclisierung der erhaltenen Verbindung und, wenn notwendig, Abspalten der Phenoxyschutzgruppe.

**2.** Verfahren nach Anspruch 1, worin die Schutzgruppe ein im Carbinolzentrum des enantiomeren Alkohols substituiertes Halogenatom ist.

**3.** Verfahren nach Anspruch 2, worin das Halogenatom ein Chloratom ist.

**4.** Verfahren nach Anspruch 3, worin das Chlorierungsmittel aus Triphenylphosphin mit Tetrachlorkohlenstoff und $SO_2Cl_2$ mit Methylenchlorid ausgewählt wird.

**5.** Verfahren nach Anspruch 1, worin das Blockiermittel die Äthergruppe ist und die Äthergruppe durch die Reaktion mit einer Verbindung der Formel RX erhalten ist, wobei X ein Halogenatom ist und R eine Aryl- oder Aralkyl- oder Alkyl- oder oder Alkoxyalkoxyalkyl- Gruppe ist, wobei jede Alkylgruppe bis zu 5 Kohlenstoffatomen aufweist, oder eine ter.-Butylsilylgruppe ist.

**6.** Verfahren nach Anspruch 5, worin R eine Methoxy-2-ethoxy-methylgruppe ist.

7. Verfahren nach Anspruch 1, worin das Blockiermittel die Estergruppe ist und die Estergruppe durch die Reaktion mit einer Verbindung der Formel RCOX oder $(RCO)_2O$ erhalten ist, wobei X ein Halogenatom ist und R eine nieder-Alkylgruppe mit bis zu 5 Kohlenstoffatomen darstellt.

8. Verfahren nach Anspruch 7, worin R eine Methylgruppe ist.

**Patentansprüche für folgenden Vertragsstaat : IT**

1. Stereospezifisches Verfahren zur Herstellung von (+) Enantiomeren von in 3-Stellung substituiertem Furo [3,4-c] Pyridins der allgemeinen Formel I :

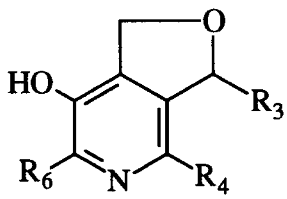

I

worin
$R_3$ einen gesättigten oder ungesättigten, geradkettigen oder verzweigten Kohlenwasserstoffrest mit 1 bis 5 Kohlenstoffatomen, eine heterocyclische Gruppe mit bis zu 6 Kohlenstoffatomen im Ring, eine Phenyl- oder Cyclohexylgruppe, eine Alkylphenyl- oder Alkenylphenylgruppe, jede dieser Gruppen gegebenenfalls substituiert mit einem oder mehreren Chlor-, Brom- oder Fluoratomen, einer oder mehreren Trifluormethylgruppen, Alkylgruppen mit 1 bis 5 Kohlenstoffatomen, Alkoxygruppen mit 1 bis 5 Kohlenstoffatomen, Alkylthiogruppen mit 1 bis 5 Kohlenstoffatomen, Dialkylaminogruppen, worin jede der Alkylgruppen 1 bis 5 Kohlenstoffatomen einschliesst, Dialkylaminoalkoxygruppen, worin jede der zwei Alkylgruppen und die Alkoxygruppe 1 bis 5 Kohlenstoffatomen enthält, oder einer $\alpha$- oder $\beta$-Alkoxy-N-Pyrrolidinylgruppe, worin das Alkoxy 1 bis 5 Kohlenstoffatomen enthält, darstellt ;
$R_4$ ein Wasserstoff- oder Halogenatom darstellt und
$R_6$ eine verzweigte oder geradkettige Alkyl- oder Alkenylgruppe mit bis zu 6 Kohlenstoffatomen, gegebenenfalls substituierten durch einen Hydroxy-, Cyano-, Amino- oder substituierten Aminorest oder durch einen Alkyl- oder Alkenylrest mit bis zu 4 Kohlenstoffatomen, darstellt, welches die folgenden Schritte umfasst :

(a) Oxidation, mit einem beliebigen Oxidationsmittel, eines razemischen Derivats eines Pyridins der allgemeinen Formel II

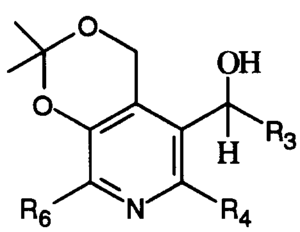

II

worin $R_3$, $R_4$ und $R_6$ wie vorstehend definiert sind, mit einem beliebigen Oxidationsmittel ;

(b) Reduktion des erhaltenen Ketons der allgemeinen Formel III

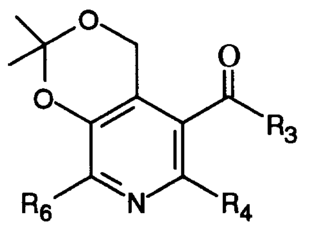

III

worin $R_3$, $R_4$ und $R_6$ wie vorstehend definiert sind, dadurch gekennzeichnet, daß ein beliebiges chirales Reduktionsmittel oder ein für eine asymmetrische Hydrierung geeigneten Katalysator verwendet werden, um die folgende Verbindung zu ergeben :

III + stereospezifisches Reduktionsmittel

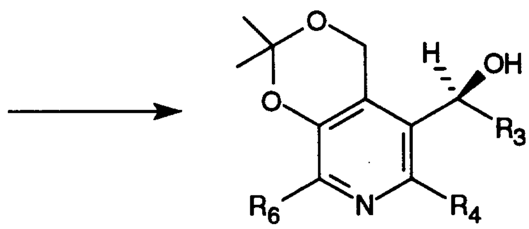

IV (+)

(c) Stereospezifisches Schützen oder Blockieren der OH-Gruppe des (+) enantiomeren Alkohols ;
(d) Öffnen des Acetonidrings mit protischen Säuren, unter gleichzeitiger Freisetzung der $CH_2OH$- und OH-Gruppe am Pyridinring ;
(e) Cyclisierung der erhaltenen Verbindung und, wenn notwendig, Abspalten der Phenoxyschutzgruppe.

**2.** Verfahren nach Anspruch 1, worin die Schutzgruppe ein im Carbinolzentrum des enantiomeren Alkohols substituiertes Halogenatom ist.

**3.** Verfahren nach Anspruch 2, worin das Halogenatom ein Chloratom ist.

**4.** Verfahren nach Anspruch 3, worin das Chlorierungsmittel aus Triphenylphosphin mit Tetrachlorkohlenstoff und $SO_2Cl_2$ mit Methylenchlorid ausgewählt wird.

**5.** Verfahren nach Anspruch 1, worin das Blockiermittel die Äthergruppe ist und die Äthergruppe durch die Reaktion mit einer Verbindung der Formel RX erhalten ist, wobei X ein Halogenatom ist und R eine Aryl- oder Aralkyl- oder Alkyl- oder Alkoxyalkoxyalkyl- Gruppe ist, wobei jede Alkylgruppe bis zu 5 Kohlenstoffatomen aufweist, oder eine ter.-Butylsilylgruppe ist.

**6.** Verfahren nach Anspruch 5, worin R eine Methoxy-2-ethoxy-methylgruppe ist.

**7.** Verfahren nach Anspruch 1, worin das Blockiermittel die Estergruppe ist und die Estergruppe durch die Reaktion mit einer Verbindung der Formel RCOX oder $(RCO)_2O$ erhalten ist, wobei X ein Halogenatom ist und R eine nieder-Alkylgruppe mit bis zu 5 Kohlenstoffatomen darstellt.

**8.** Verfahren nach Anspruch 7, worin R eine Methylgruppe ist.

**9.** (+) Enantiomere von in 3-Stellung substituiertem Furo [3,4-c] Pyridin der Formel I,

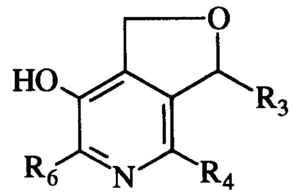

I

hergestellt nach einem der Ansprüche 1 bis 8, worin
$R_3$ einen gesättigten oder ungesättigten, geradkettigen oder verzweigten Kohlenwasserstoffrest mit 1 bis 5 Kohlenstoffatomen, eine heterocyclische Gruppe mit bis zu 6 Kohlenstoffatomen im Ring, eine Phenyl- oder Cyclohexylgruppe, eine Alkylphenyl- oder Alkenylphenylgruppe, jede dieser Gruppen gegebenenfalls substituiert mit einem oder mehreren Chlor-, Brom- oder Fluoratomen, einer oder mehreren Trifluormethylgruppen, Alkylgruppen mit 1 bis 5 Kohlenstoffatomen, Alkoxygruppen mit 1 bis 5 Kohlenstoffatomen, Alkylthiogruppen mit 1 bis 5 Kohlenstoffatomen, Dialkylaminogruppen, worin jede der Alkylgruppen 1 bis 5 Kohlenstoffatomen einschliesst, Dialkylaminoalkoxygruppen, worin jede der zwei Alkylgruppen und die Alkoxygruppe 1 bis 5 Kohlenstoffatomen enthält, oder einer $\alpha$- oder $\beta$-Alkoxy-N-Pyrrolidinylgruppe, worin das Alkoxy 1 bis 5 Kohlenstoffatomen enthält, darstellt ;
$R_4$ ein Wasserstoff- oder Halogenatom darstellt und
$R_6$ eine verzweigte oder geradkettige Alkyl- oder Alkenylgruppe mit bis zu 6 Kohlenstoffatomen, gegebenenfalls substituierten durch einen Hydroxy-, Cyano-, Amino- oder substituierten Aminorest oder durch einen Alkyl- oder Alkenylrest mit bis zu 4 Kohlenstoffatomen, darstellt,
oder Mischungen der (+) und (-) Enantiomere, worin das (+) Enantiomer überwiegt.

**10.** (+) Enantiomere von in 3-Stellung substituiertem Furo [3,4-c] Pyridin nach Anspruch 9, worin $R_3$ p-Chlorphenyl darstellt, $R_4$ H darstellt und $R_6$ $CH_3$ darstellt, sowie Mischung der (+) und (-) Enantiomere dieser Verbindung, in denen das (+) Enantiomer überwiegt.

**11.** Therapeutische Zusammensetzungen, deren wirksamer Bestandteil eine genügende Menge einer Verbindung nach Anspruch 9 ist, zusammen mit einem geeigneten Exzipienten oder Verdünnungsmittel.

**12.** Therapeutische Zusammensetzungen nach Anspruch 11, worin, pro Einheitdosis, der wirksamen Bestandteil aus 5 bis 100 mg des (+)-3-(4-Chlorphenyl)-1,3-dihydro-7-hydroxy-6-methylfuro-[3,4-c] Pyridin besteht, oder aus einer Mischung der (+) und (-) Enantiomere des 3-(4-Chlorphenyl)-1,3-dihydro-7-hydroxy-6-methylfuro-[3,4-c] Pyridins, in welcher das (+) Enantiomer überwiegt.